# EUROPÄISCHE PATENTSCHRIFT

(11) **EP 2 576 533 B1**
(45) Veröffentlichungstag und Bekanntmachung des Hinweises auf die Patenterteilung: **13.12.2017**
(21) Anmeldenummer: 11721542.6
(22) Anmeldetag: 26.05.2011
(51) Int. Cl.: C07D 309/10

(54) **VERFAHREN ZUR HERSTELLUNG VON IN 2-STELLUNG SUBSTITUIERTEN TETRAHYDROPYRANOLEN**
METHOD FOR THE PRODUCTION OF TETRAHYDROPYRANOLS SUBSTITUTED IN THE 2-POSITION
PROCÉDÉ DE PRÉPARATION DE TÉTRAHYDROPYRANOLS SUBSTITUÉS EN POSITION 2

(30) Priorität: 27.05.2010 EP 10164114
(43) Veröffentlichungstag der Anmeldung: 10.04.2013
(73) Patentinhaber: BASF SE, 67056 Ludwigshafen am Rhein (DE)
(72) Erfinder: GRALLA, Gabriele, 68161 Mannheim (DE); PELZER, Ralf, 37699 Fürstenberg (DE)
(74) Vertreter: BASF IP Association
(86) Internationale Anmeldenummer: PCT/EP2011/058646
(87) Internationale Veröffentlichungsnummer: WO 2011/147919

(56) Entgegenhaltungen:
- EP-A1- 1 493 737
- WO-A1-2010/133473
- JP-A- 2007 154 069
- SU-A1- 825 528
- PANKAJ GUPTA, VIJAY K. SETHI, SUBHASH C. TANEJA ET AL.: "Odoriferous cyclic ethers via co-halogenation reaction: facile preparation of nerol oxide, florol, florol methyl ether, and pytyol methyl ether", HELVETICA CHIMICA ACTA, Bd. 90, 2007, Seiten 196-204, XP002661260,
- ALEXANDRA MACEDO, EDISON P. WENDLER, ALCINDO A. DOS SANTOS ET AL.: "Solvent-free catalyzed synthesis of tetrahydropyran odorants: the role of SiO2.p-TSA catalyst on the Prins-cyclization reaction", J. BRAZ. CHEM. SOC., Bd. 21, Nr. 8, 4. Mai 2010 (2010-05-04), Seiten 1563-1571, XP002661261,

## Beschreibung

Die vorliegende Erfindung betrifft ein Verfahren zur Herstellung von 2-substituierten 4-Hydroxy-4-methyl-tetrahydropyranen durch Umsetzung von 3-Methylbut-3-en-1-ol (Isoprenol) mit den entsprechenden Alkenaldehyden in Gegenwart eines stark sauren Ionenaustauschers und anschliessender Hydrierung. Speziell betrifft die vorliegende Erfindung ein entsprechendes Verfahren zur Herstellung von 2-Isobutyl-4-hydroxy-4-methyl-tetrahydropyran durch Umsetzung von Isoprenol mit Prenal, und anschliessender Hydrierung.

In Tetrahedron Letters No. 51, Seiten 4507 - 4508, 1970 wird die Reaktion von 3-Alken-1-olen mit Aldehyden und deren Anwendung zur Herstellung der Aromachemikalien Rosenoxid bzw. Dihydrorosenoxid beschrieben. Dabei wird auch die Umsetzung von 3-Methylbutanal mit Isoprenol unter sauren Bedingungen erwähnt.

Die SU 825 528 offenbart ein Verfahren zur Herstellung von Di- bzw. Tetrahydropyranen und Tetrahydropyranolen durch Umsetzung von 2-Methyl-buten-1-ol-4 (Isoprenol) mit Aldehyden oder Ketonen in Gegenwart eines sauren Katalysators, wobei der saure Katalysator in einer Menge von 0,0001 bis 0,01 Gew.-% bezogen auf die Menge an Isoprenol eingesetzt wird und die Umsetzung bei einer Temperatur von 0 bis 25°C in einem organischen Losungsmittel durchgeführt wird. Als Katalysatoren werden der lonenaustauscherharz KU-2 (sulfoniertes Polystyrolharz), para-Toluolsulfonsäure, Schwefelsäure oder Perchlorsäure genannt. Beispielhaft wird u.a. die Umsetzung von Isoprenol mit Isobutyraldehyd in Gegenwart von KU-2 beschrieben.

EP 1 493 737 A1 offenbart ein Verfahren zur Herstellung von Gemischen von ethylenisch ungesättigten 4-Methyl- bzw. 4-Methylenpyranen und den entsprechenden Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d.h. der Aldehyd im Überschuss eingesetzt wird. Darüber hinaus offenbart das Dokument die anschließende Dehydrierung der genannten Gemische zu den gewünschten ethylenisch ungesättigten Pyranen. Als geeignete Katalysatoren für den ersten Reaktionsschritt werden Mineralsauren wie Salzsäure oder Schwefelsäure, bevorzugt jedoch Methansulfonsäure oder para-Toluolsulfonsäure genannt.

JP 2007-154069 betrifft 2-substituierte 4-Hydroxytetrahydropyrane mit einem Gehalt des cis-Diastereomeren von 70 bis 95 Gew.-%. Das Dokument offenbart darüber hinaus ein Verfahren zur Herstellung derselben, durch Umsetzung von Isoprenol mit einem entsprechenden Aldehyd in Gegenwart einer wässrigen Lösung eines sauren Katalysators. Dabei muss die Umsetzung bei einer Konzentration der wässrigen Katalysatorlösung entweder im Bereich von 1 bis 10 Gew.-% bei einer Temperatur von 0 bis 100°C durchgeführt werden oder im Bereich von 10 Gew.-% oder darüber bei einer Temperatur von 0 bis 30°C. Als mögliche saure Katalysatoren werden allgemein auch lonenaustauscherharze genannt. Helvetica Chimica Acta Vol. 90, 196-204, 2007 beschreibt die Herstellung des 2-substituierten 4-Hydroxy-4-methyltetrahydropyrans Florol® aus einem Geraniol/Nerol-Gemisch. Im letzten Syntheseschritt wird die Seitenkette in Position 2 hydriert.

Ausgehend von diesem Stand der Technik bestand die Aufgabe der vorliegenden Erfindung darin, ein Verfahren zur Herstellung von 2-substituierten 4-Hydroxy-4-methyl-tetrahydropyranen insbesondere von 2-Isobutyl-4-Hydroxy-4-methyl-tetrahydropyran (=Pyranol) bereitzustellen, das sich auf verfahrenstechnisch gut handhabbare Weise und mit hoher Gesamtausbeute bei möglichst hoher Chemoselektivität im technischen Maßstab durchführen lässt. Dabei sollen wohlfeile, leicht wiederzugewinnende und gut wieder einsetzbare Ausgangsverbindungen und Reagenzien bzw. Katalysatoren genutzt werden können.

Die Aufgabe wurde in überraschender Weise erfindungsgemäß gelöst durch die Bereitstellung eines Verfahrens zur Herstellung von 2-substituierten 4-Hydroxy-4-methyl-tetrahydropyranen der Formel (I) 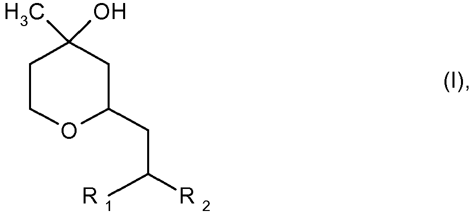 wobei der Rest
- R₁: ein geradkettiger oder verzweigter Alkylrest mit 1 bis 5 Kohlenstoffatomen ist,
- R₂: Wasserstoff oder ein geradkettiger oder verzweigter Alkylrest mit 1 bis 3 Kohlenstoffatomen ist,
umfassend die Umsetzung von 3-Methylbut-3-en-1-ol der Formel (II) 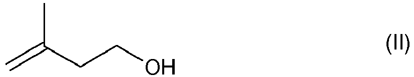 mit einem Aldehyd der Formel (III) 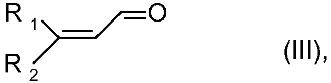 wobei die Reste R₁ und R₂ unabhängig voneinander die gleichen Bedeutungen wie in Formel (I) angegeben haben in Gegenwart von Wasser und in Gegenwart eines stark sauren Kationenaustauschers unter Bildung der Verbindung der Formel (IV), 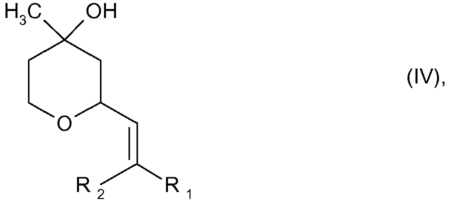 wobei die Reaktion in Gegenwart von 100 bis 200 mol-% Wasser durchgeführt wird, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe auf die Stoffmenge eines der beiden bezieht, und Hydrierung der Verbindung der Formel (IV) in Gegenwart eines Katalysators zu einer Verbindung der Formel (I).

Als Ausgangsstoffe zur Durchführung des erfindungsgemäßen Verfahrens dienen 3-Methylbut-3-en-1-ol (Isoprenol) der Formel (II), 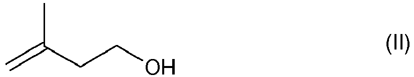 das nach bekannten Verfahren aus Isobuten und Formaldehyd in jedem Maßstab gut zugänglich und kommerziell gut verfügbar ist. An die Reinheit, Qualität oder Herstellverfahren des erfindungsgemäß einzusetzenden Isoprenols sind keine besonderen Anforderungen zu stellen. Es kann in handelsüblicher Qualität und Reinheit mit gutem Erfolg als Ausgangsstoff im Rahmen des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Als weiterer Ausgangsstoff zur Durchführung des erfindungsgemäßen Verfahrens dient ein Alkenaldehyd der Formel (III) 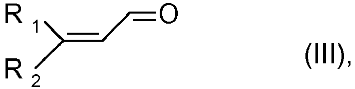 wobei der Rest R₁ für einen geradkettigen oder verzweigten Alkylrest mit 1 bis 5 Kohlenstoffatomen und R₂ für Wasserstoff oder einen geradkettigen oder verzweigten Alkylrest mit 1 bis 3 Kohlenstoffatomen stehen kann.

Unter einem Alkylsubstituenten R₁ ist ein solcher zu verstehen der 1 bis 5 Kohlenstoffatome aufweist wie beispielsweise Methyl, Ethyl, n-Propyl, iso-Propyl, n-Butyl, sec-Butyl, tert.-Butyl oder n-Pentyl, bevorzugt Methyl, Ethyl, n-Propyl, iso-Propyl, und ganz besonders bevorzugt Methyl.

Unter einem Alkylsubstituenten R₂ ist ein solcher zu verstehen der 1 bis 3 Kohlenstoffatome aufweist wie beispielsweise Methyl, Ethyl, n-Propyl oder iso-Propyl, bevorzugt Methyl oder Ethyl, und ganz besonders bevorzugt Methyl.

In dem erfindungsgemässen Verfahren bedeuten die Worte "stark saurer Kationenaustauscher", dass der Ionenaustauscher in H⁺-Form vorliegt.

Erfindungsgemäß ganz besonders bevorzugte Alkenaldehyde der Formel (III) sind solche, bei denen der Rest R₁ für einen geradkettigen oder verzweigten Alkylrest mit 1 bis 3 Kohlenstoffatomen und der Rest R₂ für einen Alkylrest mit 1 bis 2 Kohlenstoffatomen steht. Erfindungsgemäß bevorzugte Bedeutungen für den Rest R₁ sind somit beispielsweise Methyl, Ethyl, n-Propyl und iso-Propyl, ganz besonders bevorzugt Methyl, und bevorzugte Bedeutungen für den Rest R₂ sind somit Methyl oder Ethyl, und ganz besonders bevorzugt Methyl. Als dementsprechend erfindungsgemäß bevorzugt einzusetzende Alkenaldehyde der Formel (III) seien genannt: Prenal (3-Methyl-2-butenal), 2-Butenal, 3-Methyl-2-pentenal, 2-Pentenal, 2-Hexenal, 3-Methyl-2-hexenal. Ein erfindungsgemäß ganz besonders bevorzugt einzusetzender Alkenaldehyde der Formel (III) ist Prenal.

Die im Rahmen des erfindungsgemäßen Verfahrens einzusetzenden Ausgangsstoffe Isoprenol und der jeweils gewählte Aldehyd der Formel (III) können in unterschiedlichen Mengenverhältnissen miteinander umgesetzt werden. So ist es möglich eine der beiden Ausgangsstoffe im Überschuss einzusetzen, wobei die Höhe des gewählten Überschusses sich in verfahrenstechnisch und wirtschaftlich vorteilhaften Grenzen bewegen sollte, ansonsten im Prinzip jedoch frei gewählt werden kann. Der Stöchiometrie der erfindungsgemäßen Umsetzung von Isoprenol mit dem gewählten Aldehyd der Formel (III) folgend, werden Isoprenol und der Aldehyd der Formel (III), bevorzugt Prenal, in einem molaren Verhältnis im Bereich von 1 zu 2 bis 2 zu 1 eingesetzt, entsprechend einem doppelten molaren Überschuss einer der Ausgangsstoffe. Im Rahmen einer bevorzugten Ausführungsform führt man das erfindungsgemäße Verfahren so durch, dass man Isoprenol und den Aldehyd der Formel (III) in einem molaren Verhältnis von 0,7 zu 1 bis 2 zu 1 einsetzt. Besonders bevorzugt führt man das erfindungsgemäße Verfahren so durch, dass man Isoprenol und den Aldehyd der Formel (III) in einem molaren Verhältnis von 1 zu 1 bis 2 zu 1 einsetzt. Ganz besonders bevorzugt führt man das erfindungsgemäße Verfahren so durch, dass man Isoprenol und den Aldehyd der Formel (III) in einem molaren Verhältnis von 1 zu 1 bis 1,5 zu 1 einsetzt.

Die im Rahmen des erfindungsgemäßen Verfahren zur Herstellung der 2-substituierten 4-Hydroxy-4-methyl-tetrahydropyranen der Formel (I), bevorzugt zur Herstellung von 2-Iso-Butyl-4-Hydroxy-4-methyl-tetrahydropyran durchzuführende Umsetzung von Isoprenol mit dem gewählten Aldehyd der Formel (III), bevorzugt mit Prenal, wird in Gegenwart von Wasser durchgeführt. Dies bedeutet, dass dem Reaktionsgemisch neben Isoprenol, dem Aldehyd der Formel (III) und dem gewählten stark sauren Kationenaustauscher auch Wasser zugesetzt wird. Zusätzlich kann das Reaktionsgemisch noch geringe Mengen Wasser enthalten, das durch die möglicherweise als unerwünschte Nebenreaktion erfolgende Dehydratisierung des gewünschten Verfahrensproduktes der Formel (I) freigesetzt werden kann.
Üblicherweise führt man die Umsetzung des Isoprenols mit dem gewählten Aldehyd der Formel (III) in Gegenwart von etwa mindestens 10 mol-% Wasser durch, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 10- bis 100-fachem Überschuss oder auch darüber eingesetzt werden. Vorzugsweise bereitet man ein Gemisch aus Isoprenol und dem gewählten Aldehyd der Formel (III), vorzugsweise Prenal, mit der gewählten Menge Wasser, so dass das zugegebene Wasser in dem Gemisch aus Isoprenol und dem gewählten Aldehyd gelöst bleibt d.h. kein zweiphasiges System vorliegt.
Üblicherweise setzt man im Rahmen des erfindungsgemäßen Verfahrens die Ausgangsstoffe Isoprenol und den gewählten Aldehyd der Formel (III) in Gegenwart von mindestens 25 mol-%, bevorzugt von mindestens 50 mol-%, noch mehr bevorzugt von mindestens 75 und noch mehr bevorzugt von mindestens 90 bis etwa 1000 mol-% Wasser um, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht. Die erfindungsgemäße Umsetzung wird in Gegenwart einer mindestens äquimolaren Menge an Wasser durchführt, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht. Demzufolge führt man die erfindungsgemäße Umsetzung von Isoprenol mit dem gewählten Aldehyd der Formel (III) in Gegenwart von 100 bis 200 mol-% und bevorzugt in Gegenwart von 100 bis 180 mol-% Wasser durch, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Die genannten Ausgangsstoffe, d.h. Isoprenol und der jeweils gewählte Aldehyd und das in der vorstehenden Menge einzusetzende Wasser können in beliebiger Reihenfolge miteinander in Kontakt gebracht bzw. gemischt werden. Üblicherweise bereitet man ein Gemisch aus Isoprenol und dem gewählten Aldehyd der Formel (III) mit der gewählten Menge Wasser und setzt dieses Gemisch im Rahmen der erfindungsgemäß durchzuführenden Umsetzung ein.

Die im Rahmen des erfindungsgemäßen Verfahrens zur Herstellung der gewünschten 2-substituierten 4-Hydroxy-4-methyl-tetrahydropyranen der Formel (I) durchzuführende Umsetzung von Isoprenol mit dem gewählten Aldehyd der Formel (III) wird in Gegenwart eines stark sauren Kationenaustauschers durchgeführt. Unter dem Begriff stark saurer Kationenaustauscher sind dabei im Rahmen der vorliegenden Erfindung solche Kationenaustauscher zu verstehen, die stark saure Gruppen, in der Regel Sulfonsäuregruppen, aufweisen, deren Matrix gelförmig bzw. makroporös sein kann.

Eine bevorzugte Ausführungsform des erfindungsgemäßen Verfahrens ist dementsprechend dadurch gekennzeichnet, dass man einen stark sauren, Sulfonsäuregruppen aufweisenden bzw. umfassenden Kationenaustauscher einsetzt.

Stark saure Kationenaustauscher sind insbesondere lonenaustauscherharze in der H+-Form. Als solche kommen beispielsweise in Betracht:
- stark saure Ionenaustauscher (wie z.B. Amberlyst, Amberlite, Dowex, Lewatit, Purolite, Serdolit), die auf Polystyrol basieren, und die Copolymere aus Styrol und Divinylbenzol als Trägermatrix mit Sulfonsäuregruppen in H+-Form enthalten,
- mit Sulfonsäuregruppen (-SO₃H) funktionalisierte Ionenaustauschergruppen.

Die Ionenaustauscher unterscheiden sich im Aufbau ihrer Polymergerüste, und man unterscheidet gelförmige und makroporöse Harze. Die stark sauren Ionentauscherharze werden in der Regel mit Salzsäure und / oder Schwefelsäure regeneriert.

Bei Nafion® handelt es sich dabei um perfluorierte lonenaustauschmaterialien bestehend aus Fluorkohlenstoff-Basisketten und perfluorierten Seitenketten, die Sulfonsäuregruppen enthalten. Die Harze werden durch eine Copolymerisation von perfluorierten, endständig ungesättigten und mit Sulfonylfluorid funktionalisierten Ethoxylaten mit Perfluorethen hergestellt. Nafion® zählt zu den gelartigen Ionenaustauscherharzen. Als Beispiel für ein solches perfluoriertes polymeres lonenaustauscherharz sein Nafion® NR-50 genannt.

Eine besonders bevorzugte Ausführungsform des erfindungsgemässen Verfahrens ist dadurch gekennzeichnet, dass man mindestens einen stark sauren Kationenaustauscher in der H+-Form einsetzt, wobei der Ionenaustauscher ein Sulfonsäuregruppen aufweisendes Polymergerüst enthält und entweder gelförmig ist oder makroporöse Harze enthält.

Eine ganz besonders bevorzugte Ausführungsform des erfindungsgemässen Verfahrens ist dadurch gekennzeichnet, dass der Ionenaustauscher auf einem Polystyrolgerüst mit Sulfonsäuregruppen oder auf einem perfluorierten lonenaustauscherharz mit Sulfonsäuregruppen basiert.

Die kommerziell verfügbaren stark sauren Kationenaustauscher sind unter den Handelsnamen) Lewatit® (Lanxess), Purolite® (The Purolite Company), Dowex® (Dow Chemical Company), Amberlite® (Rohm and Haas Company), Amberlyst™ (Rohm and Haas Company) bekannt.

Als erfindungsgemäß bevorzugte stark saure Kationenaustauscher seien beispielsweise genannt: Lewatit® K 1221, Lewatit® K 1461, Lewatit® K 2431, Lewatit® K 2620, Lewatit® K 2621, Lewatit® K 2629, Lewatit® K 2649, Amberlite® IR 120, Amberlyst™ 131, Amberlyst™ 15, Amberlyst™ 31, Amberlyst™ 35, Amberlyst™ 36, Amberlyst™ 39, Amberlyst™ 46, Amberlyst™ 70, Purolite® SGC650, Purolite® C100H, Purolite® C150H, Dowex® 50X8, Serdolit® red und Nafion® NR-50.

Im Rahmen einer bevorzugten Ausführungsform führt man die erfindungsgemäß durchzuführende Umsetzung von Isoprenol mit dem gewählten Aldehyd der Formel (III) in Gegenwart mindestens eines stark sauren Kationenaustauschers durch, der ausgewählt ist aus der Gruppe der Kationenaustauscher umfassend Lewatit® K 1221, Lewatit® K 2629, Amberlyst™ 131, Purolite® SGC650, Purolite® C100H, Purolite® C150H, Amberlite® IR 120 und Dowex® 50X8.

Erfindungsgemäß insbesondere bevorzugte stark saure Kationenaustauscher sind die Kationenaustauscher Amberlyst™ 131 und/oder Lewatit® K 1221.

Ein erfindungsgemäß ganz besonders bevorzugter stark saurer Kationenaustauscher ist Amberlyst™ 131, der wie die anderen genannten Kationenaustauscher kommerziell verfügbar ist.

Zur Durchführung der erfindungsgemäßen Umsetzung von Isoprenol mit dem Aldehyd der Formel (III) bringt man die genannten Ausgangsstoffe und die gewählte Menge Wasser, vorzugsweise in Form eines Gemisches, mit dem gewählten stark sauren Kationenaustauscher in Kontakt. Die Menge an einzusetzendem Kationenaustauscher ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des gewählten stark sauren Kationenaustauschers durchgeführt werden. Üblicherweise setzt man den gewählten Kationentauscher in einer Menge von etwa 5 bis etwa 40 Gew.-%, bevorzugte in einer Menge von etwa 20 bis etwa 40 Gew.-% und besonders bevorzugt in einer Menge von etwa 20 bis etwa 30 Gew.-%, jeweils bezogen auf die Summe an eingesetztem Isoprenol und Aldehyd der Formel (III), ein. Dabei beziehen sich die Angaben auf den gebrauchsfertigen Kationenaustauscher, der in der Regel mit Wasser vorbehandelt wird und dementsprechend Mengen von bis zu etwa 70 Gew.%, bevorzugt von etwa 30 bis etwa 65 Gew-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen.

Die genannten stark sauren Kationenaustauscher können sowohl einzeln oder auch in Form von Gemischen untereinander im Rahmen des erfindungsgemäßen Verfahrens eingesetzt werden.

Die erfindungsgemäß durchzuführende Umsetzung kann wahlweise auch in Gegenwart eines unter den Reaktionsbedingungen inerten Lösungsmittels wie beispielsweise tert-Butylmethylether, Cyclohexan, Toluol, Hexan oder Xylol durchgeführt werden. Die genannten Lösungsmittel können alleine oder in Form von Gemischen untereinander eingesetzt werden. Im Rahmen einer bevorzugten Ausführungsform des erfindungsgemäßen Verfahrens führt man die Umsetzung von Isoprenol mit dem gewählten Aldehyd der Formel (III) ohne Zusatz eines organischen Lösungsmittels durch.

Die erfindungsgemäß durchzuführende Umsetzung von Isoprenol mit dem gewählten Aldehyd der Formel (III) in Gegenwart von Wasser und in Gegenwart eines stark sauren Kationentauschers wird üblicherweise bei einer Temperatur im Bereich von 0 bis 100°C, bevorzugt bei einer Temperatur im Bereich von 20 bis 90°C und besonders bevorzugt bei einer Temperatur im Bereich von 20 bis 80°C durchgeführt, wobei sich die Temperatur auf die des Reaktionsgemisches bezieht.

Die erfindungsgemäß durchzuführende Umsetzung kann wahlweise diskontinuierlich oder kontinuierlich durchgeführt werden. Dabei kann man beispielsweise im diskontinuierlichen Fall die Umsetzung so vornehmen, dass man ein Gemisch von Isoprenol, dem gewählten Aldehyd der Formel (III) und Wasser in einem geeigneten Reaktionsgefäß vorlegt und den stark sauren Kationenaustauscher zugibt. Nach Abschluss der Reaktion kann dann der Kationenaustauscher durch geeignete Trennverfahren, vorzugsweise durch Filtration oder auch durch Zentrifugation, vom erhaltenen Reaktionsgemisch abgetrennt werden. Die Reihenfolge des Inkontaktbringens der einzelnen Reaktionskomponenten ist nicht kritisch und kann nach Maßgabe der jeweiligen verfahrenstechnischen Ausgestaltung variiert werden.

Im Rahmen einer bevorzugten Ausführungsform führt man die erfindungsgemäß durchzuführende Umsetzung von Isoprenol mit dem gewählten Aldehyd der Formel (III) kontinuierlich durch. Dazu kann beispielsweise eine Mischung der umzusetzenden Ausgangsstoffe Isoprenol und Aldehyd der Formel (III) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und die Ausgangsstoffe und das Wasser in diesen kontinuierlich ausgetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden.

Der Hydrierungsschritt des erfindungsgemäßen Verfahrens wird vorzugsweise in Gegenwart von Wasserstoff und in Gegenwart eines heterogenen Katalysators durchgeführt, wobei der einzusetzende heterogene Katalysator 30 bis 70 Gew.-%, bevorzugt 40 bis 60 Gew.-% sauerstoffhaltige Verbindungen des Nickels, berechnet als NiO, 15 bis 45 Gew.-%, bevorzugt 20 bis 40 Gew.-% sauerstoffhaltige Verbindungen des Zirkons, berechnet als ZrO₂, 5 bis 30 Gew.-%, bevorzugt 10 bis 25 Gew.-% sauerstoffhaltige Verbindungen des Kupfers, berechnet als CuO und 0,1 bis 10 Gew.-%, bevorzugt 0,5 bis 5 Gew.-% sauerstoffhaltige Verbindungen des Molybdäns, berechnet als MoO₃ gegebenenfalls neben weiteren Komponenten in einer Menge von 0 bis 10 Gew.-%, bevorzugt 0 bis 5 Gew.-% wie beispielsweise Graphit enthält. Dabei beziehen sich die Angaben in Gew.-% auf den trockenen, nicht reduzierten Katalysator.

Im Rahmen einer bevorzugten Ausführungsform des erfindungsgemässen Verfahrenssetzt man zur Durchführung solche Katalysatoren ein, umfassend 45 bis 55 Gew.-% sauerstoffhaltige Verbindungen des Nickels, berechnet als NiO, 25 bis 35 Gew.-% sauerstoffhaltige Verbindungen des Zirkons, berechnet als ZrO₂, 5 bis 20 Gew.-% sauerstoffhaltige Verbindungen des Kupfers, berechnet als CuO, - 0,1 bis 3 Gew.-% insbesondere 1 bis 3 Gew.-% sauerstoffhaltige Verbindungen des Molybdäns, berechnet als MoO₃
0 bis 5 Gew.-% weiterer Komponenten,
wobei sich die Angaben in Gew.-% zu 100 Gew.-% ergänzen und sich auf den trockenen, nicht reduzierten Katalysator beziehen. Erfindungsgemäß insbesondere bevorzugt sind solche Katalysatoren, die aus den vorstehend genannten Komponenten in den ebenfalls vorstehend genannten Gewichtsanteilen bestehen.

Ein zum Einsatz im Rahmen des erfindungsgemäßen Verfahrens insbesondere bevorzugter Katalysator besteht zu 49 bis 53 Gew.-% aus NiO, zu 15 bis 19 Gew.-% aus CuO, zu 28 bis 32 Gew.-% aus ZrO₂ und zu 1 bis 2 Gew.-% aus MoO₃ sowie gegebenenfalls zu 0 bis 3 Gew.-% aus weiteren Komponenten wie beispielsweise Graphit, wobei sich die jeweils gewählten Gewichtsanteile der einzelnen Komponenten auf den trockenen, nicht reduzierten Katalysator beziehen und zu 100 Gew.-% ergänzen. Derartige Katalysatoren sind bekannt und können beispielsweise wie in der EP 0 696 572 beschrieben hergestellt werden.

Die erfindungsgemäß einsetzbaren Katalysatoren können z.B. durch Einsatz von Fällungsmethoden hergestellt werden. So können sie beispielsweise durch eine gemeinsame Fällung der Nickel- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -Silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkonium-Salzlösungen mittels Mineralbasen erhalten.

Bevorzugt werden die erfindungsgemäß einsetzbaren Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.
Erfindungsgemäß einsetzbare Katalysatoren mit besonders vorteilhaften Eigenschaften sind dadurch erhältlich, dass man einen Teil der Zirkoniumkomponente des Katalysators, zweckmäßigerweise aus einer wässrigen Zirkoniumsalzlösung, separat in einer Fällungsapparatur durch Zugabe wässriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig 10 bis 80 Gew.-%, vorzugsweise 30 bis 70 Gew.-% und insbesondere 40 bis 60 Gew.-% der Gesamtzirkoniummenge der katalytisch aktiven Masse vorzufällen.

Die auf diese Weise hergestellten Katalysatoren können als solche gelagert und eingesetzt werden. Vor ihrem Einsatz als Katalysatoren im Rahmen des erfindungsgemäßen Verfahrens werden sie üblicherweise vorreduziert. Sie können jedoch auch ohne Vorreduktion eingesetzt werden, wobei sie dann unter den Bedingungen der erfindungsgemäßen Hydrierung durch den im Reaktor vorhandenen Wasserstoff reduziert werden. Zur Vorreduktion werden die Katalysatoren im allgemeinen zunächst bei 150 bis 200° C über einen Zeitraum von 12 bis 20 Stunden einer Stickstoff-Wasserstoff- Atmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen üblicherweise zu den entsprechenden Metallen reduziert, so dass diese gemeinsam mit den verschiedenartigen Sauerstoffverbindungen in der aktiven Form des Katalysators vorliegen.
Im Allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Kugeln, Zylinder, Tabletten, Ringe, Spiralen, Stränge und dergleichen mehr - im Reaktor anordnet.
Im Rahmen einer weiteren Ausführungsform des erfindungsgemäßen Hydrierverfahrens setzt man den gewählten heterogenen Katalysator in Form eines Festbettkatalysators ein.
Zur Durchführung des erfindungsgemäßen Verfahrens bringt man die wie vorstehend beschriebenene Verbindung der Formel (IV) mit Wasserstoff und dem gewählten Katalysator in Kontakt. Der Wasserstoff kann dabei in unverdünnter Form, üblicherweise in einer Reinheit von etwa 99,9 Vol.-% oder in verdünnter Form, d.h. in Form von Gemischen mit inerten Gasen wie beispielsweise Stickstoff oder Argon, eingesetzt werden. Bevorzugt setzt man Wasserstoff in unverdünnter Form ein.
Die Umsetzung kann mit gutem Erfolg mit oder ohne Zusatz eines Lösungsmittels erfolgen. Für den Fall, dass die Umsetzung in Gegenwart eines Lösungsmittels erfolgt, kommen unter den Reaktionsbedingungen inerte organische Lösungsmittel in Betracht, wie beispielsweise Methanol, Ethanol, Isopropanol, Hexan, Heptan, Cyclohexan und dergleichen. Bevorzugt führt man die Reaktion in Methanol als Lösungsmittel durch.

Die erfindungsgemäße Hydrierung kann bei einem Wasserstoffdruck (absolut) im Bereich von 1 bis 200 bar, bevorzugt von 2 oder besser von 3 bis 200 bar, besonders bevorzugt von 4 oder 5 bis 150 bar, besonders bevorzugt von 5 bis 100 bar und ganz besonders bevorzugt im Bereich von 5 bis 50 bar durchgeführt werden. Als Reaktionstemperatur zur Durchführung der erfindungsgemäßen Hydrierung wählt man vorteilhaft eine Temperatur im Bereich von 20 bis 150°C, bevorzugt von 40 bis 130°C, besonders bevorzugt von 60 bis 110°C und ganz besonders bevorzugt von 70 bis 100°C. Praktisch geht man bei der Durchführung im allgemeinen so vor, dass man dem Katalysator, der sich üblicherweise in einem bevorzugt von außen beheizten Festbettreaktor wie beispielsweise einem Rohrreaktor, Autoklaven oder Rohrbündelreaktor befindet, bei der gewünschten Reaktionstemperatur und dem gewünschten Druck das umzusetzende Produkt der Formel (IV) zuführt. Dabei belastet man den Katalysator im Allgemeinen mit 0,1 bis 1,0, bevorzugt mit 0,1 bis 0,6 und besonders bevorzugt mit 0,2 bis 0,4 kg der Verbindung der Formel (IV) pro kg Katalysator und pro Stunde. Hierbei kann es zweckmäßig sein, das einzusetzende Produkt der Formel (IV) bereits vor der Zuführung in das Reaktionsgefäß bzw. den Reaktor zu erwärmen und zwar bevorzugt auf die Reaktionstemperatur.

Das erfindungsgemäße Hydrierverfahren kann sowohl diskontinuierlich als auch kontinuierlich durchgeführt werden. In beiden Fällen kann nicht umgesetztes Edukt zusammen mit dem Wasserstoff im Kreis geführt werden.

Eine bevorzugte Ausführungsform des erfindungsgemäßen Verfahrens betrifft dementsprechend ein kontinuierliches Verfahren zur Herstellung von 2-substituierten 4-Hydroxy-4-methyl-tetrahydropyranen der Formel (I) umfassend die Schritte
a. Bereitstellen eines den gewählten stark sauren Kationenaustauscher enthaltenden Durchflussreaktors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (III) sowie Wasser mit dem stark sauren Kationenaustauscher im Durchflussreaktor unter Erhalt eines die gewünschte Verbindung der Formel (IV) umfassenden Reaktionsgemisches
d. kontinuierliche Hydrierung des die Verbindung der Formel (IV) umfassenden Reaktionsgemisches, und
e. kontinuierliches Austragen des Reaktionsgemisches.

Der gewählte stark saure Kationenaustauscher kann dabei sowohl in Form einer losen Schüttung als auch in Form eines Festbettes im vorstehend genannten Durchflussreaktor vorliegen.

Es ist auch möglich die erfindungsgemäß durchzuführende Umsetzung von Isoprenol mit dem Aldehyd der Formel (III) in einer Kaskade von mehreren, beispielsweise 2 oder 3 hintereinander geschalteten Durchflussreaktoren durchzuführen, wobei die einzelnen Durchflussreaktoren auch mit verschiedenen stark sauren Kationenaustauschern befüllt sein können und im Fall des Einsatzes von Rohrreaktoren, diese sowohl in Sumpf als auch in Rieselfahrweise betrieben werden können. Darüber hinaus kann das aus dem gewählten Durchflussreaktor ausgetragene Reaktionsgemisch gewünschtenfalls auch teilweise wieder in die kontinuierlichbetriebene Umsetzung zurückgeführt werden.

Das erfindungsgemäße Verfahren ermöglicht die Herstellung von 2-substituierten 4-Hydroxy-4-methyl-tetrahydropyranen der Formel (I), speziell die Herstellung von 2-lso-Butyl-4-Hydroxy-4-methyl-tetrahydropyranen der Formel (I). Diese fallen üblicherweise in Form von Reaktionsgemischen an, die neben den gewünschten Zielverbindungen, noch Reste der eingesetzten Ausgangsstoffe, das eingesetzte Wasser sowie möglicherweise in geringem Ausmaß auch die dehydratisierten Nebenprodukte der Formeln (Va) und (Vb) - 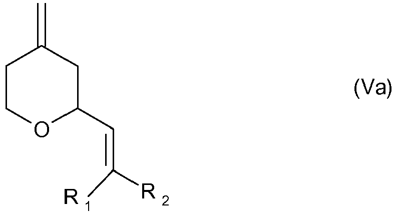 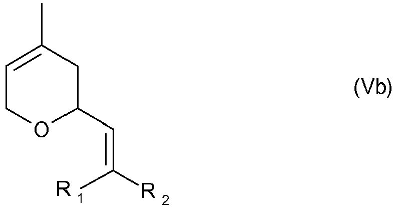 enthalten können, wobei R₁ und R₂ die unter Formel (I) angegebenen Bedeutungen haben können. Das erfindungsgemäße Verfahren ermöglicht die Herstellung der gewünschten Hydroxypyrane der Formel (I) bzw. bevorzugt des 2-Iso-butyl-4-hydroxy-4-methyltetrahydropyrans in hoher Ausbeute und hoher Reinheit, wobei die unerwünschten Dehydratisierungsprodukte der Formeln (Va) bis (Vb) wenn überhaupt, nur in untergeordnetem Ausmaß anfallen.

Diese Nebenprodukte können, genauso wie die nicht umgesetzten bzw. im Überschuss eingesetzten Ausgangsverbindungen in vorteilhafter Weise wieder in die Reaktion zurückgeführt werden.

Die erfindungsgemäß erhaltenen Reaktionsgemische bestehen typischerweise zu etwa 50 bis zu etwa 90 Gew.-%, häufig zu etwa 60 bis zu etwa 80 Gew.-% aus den gewünschten 2-substituierten 4-Hydroxy-4-methyl-tetrahydropyranen der Formel (I) und nur bis zu etwa 20 Gew.-%, bevorzugt nur bis zu etwa 15 Gew.-% und besonders bevorzugt nur bis zu 10 Gew.-% aus den Dehydratisierungsprodukten der Formeln (IVa) bis (IVc), jeweils bezogen auf das Gesamtgewicht des erhaltenen Rohproduktes und im übrigen aus den nicht umgesetzten bzw. im Überschuss eingesetzten Ausgangsstoffen sowie den anderen genannten Nebenprodukten.

Die als Rohprodukt erhaltenen Stoffgemische lassen sich gut nach dem Fachmann bekannten Verfahren, insbesondere durch Destillation bzw. Rektifikation, weiter aufreinigen. Auf diese Weise erhält man das jeweils gewünschte 2-substituierte 4-Hydroxy-4-methyl-tetrahydropyran der Formel (I), insbesondere bei Einsatz von Isoprenol und Prenal und nachfolgender Hydrierung das gewünschte 2-Iso-butyl-4-hydroxy-4-methyltetrahydropyran in einer Reinheit von über 95 Gew.-% oder bevorzugt von 97 bis 99,9 Gew.-% oder besonders bevorzugt von 98 bis 99,8 Gew.-% , d.h. in einer Qualität, wie sie beispielsweise für den Einsatz als Aromachemikalie erforderlich ist.

Eine bevorzugte Ausführungsform des erfindungsgemäßen Verfahrens betrifft die Herstellung von 2-substituierten 4-Hydroxy-4-methyl-tetrahydropyranen in Form von Gemischen der cis-Diastereomeren der Formel (Ib) 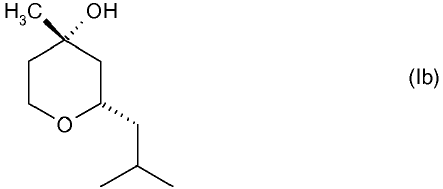 und der trans-Diastereomeren der Formel (Ic) 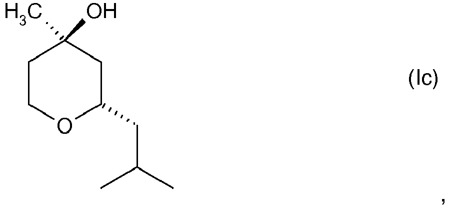 wobei das Diastereomerenverhältnis des cis-Diasteromeren der Formel (Ib) zum trans-Diastereomeren der Formel (Ic) 65 zu 35 bis 95 zu 5, bevorzugt 70 zu 30 bis 85 zu 15 beträgt.

Insbesondere für die erfindungsgemäß bevorzugte Umsetzung von Isoprenol mit Prenal und nachfolgender Hydrierung erhält man im Rahmen des erfindungsgemäßen Verfahrens 2-Isobutyl-4-hydroxy-4-methyl-tetrahydropyran in Form von Gemischen des cis-Diastereomeren der Formel (Ib) 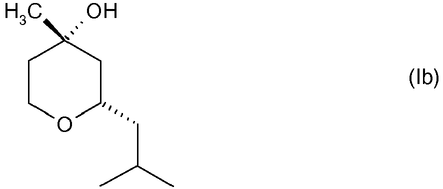 und der trans-Diastereomeren (Ic) 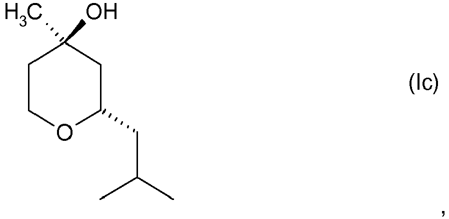 wobei das Diastereomerenverhältnis des cis-Diasteromeren der Formel (Ib) zum trans-Diastereomeren der Formel (Ic) 65 zu 35 bis 95 zu 5, bevorzugt 70 zu 30 bis 85 zu 15 beträgt. Derartige Gemische eignen sich aufgrund ihrer besonderen GeruchsEigenschaften in besonderem Maße zur Verwendung als Aromachemikalien, beispielsweise als Komponente mit Maiglöckchenduft zur Herstellung von Riechstoffkompositionen.

Die vorliegende Erfindung betrifft daher im Rahmen einer bevorzugten Ausführungsform ein Verfahren zur Herstellung von 2-Isobutyl-4-hydroxy-4-methyl-tetrahydropyran der Formel (la) 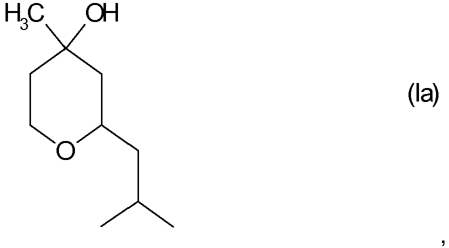 umfassend die Umsetzung von 3-Methylbut-3-en-1-ol der Formel (II) mit Prenal der Formel (IIIa) 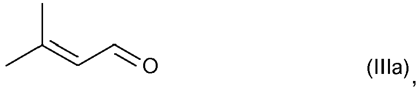 in Gegenwart von Wasser und in Gegenwart eines stark sauren Kationenaustauschers unter Bildung des Produkts der Formel (IVa) 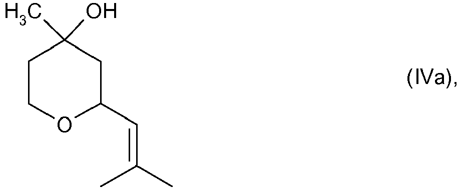 und nachfolgender katalytischen Hydrierung in Gegenwart von Wasserstoff und einem Nickel-haltigen Katalysator unter Bildung der Verbindung der Formel (la).

Die folgenden Beispiele dienen der Erläuterung der Erfindung, ohne sie in irgendeiner Weise zu beschränken:
Gaschromatographische Analysen wurden nach der folgenden Methode durchgeführt: 30 m DB-WAX, ID.: 0,32 mm, FD.: 1,2 µm; 50°C, 3°C/min - 170 °C- 20 °C/min auf 230 °C; t*_{R}* = min; Trägergas: He; Probe: 0.2 µl; t*_{R}* (Prenal): 9,1; t*_{R}* (Isoprenol): 10,6; t*_{R}* (Dehydrorosenoxid der Formel (Va)): 15,6; t*_{R}* (Neroloxid der Formel (Vb)): 18,5; t*_{R}* (trans-Pyranol der Formel (Ic)): 28,5; t*_{R}* (cis-Pyranol der Formel (Ib)): 29,8; t*_{R}* (trans-Hydroxyrosenoxid der Formel IVa): 34,2; t*_{R}* (cis-Hydroxyrosenoxid der Formel IVa): 35,4; t*_{R}* (2-(2-Hydroxymethylpropyl)-4-methyltetrahydropyranol): 41,5 und 42,2.

### Beispiel 1: Herstellung von trans- und cis-Hydroxyrosenoxid

Der Ionenaustauscher wurde vor dem Einsatz zunächst mehrmals mit Wasser gewaschen, dann einmal mit Methanol und abschließend mit Wasser Methanol-frei gewaschen.

In einem Kolben wurden 1.7 g Amberlyst™ 131 (58 Gew.% H₂O) und 4.2 g (0.05 mol) Isoprenol bei Raumtemperatur vorgelegt und anschließend wurden 4.3 g (0.05 mol) Prenal zugetropft. Das Reaktionsgemisch wurde 3 h bei Raumtemperatur gerührt. Das abreagierte Reaktionsgemisch wurde mit 30 ml MTBE versetzt und der Ionenaustauscher wurde anschließend abfiltriert. Der Ionenaustauscher wurde zweimal mit je 5 ml MTBE gewaschen. Die erhaltene Reaktionslösung wurde gaschromatographisch analysiert (in GC-Flächen-%). Das gelbe Filtrat wurde am Rotationsverdampfer eingeengt und man erhielt 7.1 g Rohprodukt.

| | |
|---|---|
| Prenal | 1,8 GC-Flächen-% |
| Isoprenol | 3,8 GC-Flächen-% |
| Dehydrorosenoxid | 1,6 GC-Flächen-% |
| Neroloxid | 4,5 GC-Flächen-% |
| trans-Hydroxyrosenoxid | 17,7 GC-Flächen-% |
| cis-Hydroxyrosenoxid | 67,5 GC-Flächen-% |

### Beispiel 2: Herstellung von trans- und cis-Hydroxyrosenoxid

In einem Kolben wurden 1.7 g (20 Gew.%) Amberlyst™ 131 (58 Gew.% H₂O) und 4.2 g (0.05 mol) Isoprenol bei Raumtemperatur vorgelegt und anschließend wurden 4.3 g (0.05 mol) Prenal zugetropft. Das Reaktionsgemisch wurde 1 h bei 60 °C gerührt. Nach Abkühlen des abreagierten Reaktionsgemisches wurde MTBE (30 ml) zugegeben und der Ionenaustauscher wurde anschließend abfiltriert. Der Ionenaustauscher wurde zweimal mit je 5 ml MTBE gewaschen. Die erhaltene Reaktionslösung wurde gaschromatographisch analysiert (in GC-Flächen-%). Das gelbe Filtrat wurde am Rotationsverdampfer eingeengt und man erhielt 7.6 g Rohprodukt.

| | |
|---|---|
| Prenal | 0,9 GC-Flächen-% |
| Isoprenol | 1,2 GC-Flächen-% |
| Dehydrorosenoxid | 1,8 GC-Flächen-% |
| Neroloxid | 4,5 GC-Flächen-% |
| trans-Hydroxyrosenoxid | 31,1 GC-Flächen-% |
| cis-Hydroxyrosenoxid | 55,5 GC-Flächen-% |
| 2-(2-Hydroxymethylpropyl)-4-methyltetrahydropyranol | 2,7 GC-Flächen-% |

### Beispiel 3: Herstellung von trans- und cis-Hydroxyrosenoxid

In einem Kolben wurden 1.7 g (20 Gew.%) Amberlyst™ 131 (58 Gew.% H₂O) und 4.2 g (0.05 mol) Isoprenol bei Raumtemperatur vorgelegt und anschließend wurden 4.3 g (0.05 mol) Prenal zugetropft. Das Reaktionsgemisch wurde 1 h bei 80 °C gerührt. Nach Abkühlen des abreagierten Reaktionsgemisches wurde MTBE (30 ml) zugegeben und der Ionenaustauscher wurde anschließend abfiltriert. Der Ionenaustauscher wurde zweimal mit je 5 ml MTBE gewaschen. Die erhaltene Reaktionslösung wurde gaschromatographisch analysiert (in GC-Flächen-%). Das gelbe Filtrat wurde am Rotationsverdampfer eingeengt und man erhielt 7.6 g Rohprodukt.

| | |
|---|---|
| Prenal | 0,4 GC-Flächen-% |
| Isoprenol | 0,3 GC-Flächen-% |
| Dehydrorosenoxid | 1.7 GC-Flächen-% |
| Neroloxid | 4,9 GC-Flächen-% |
| trans-Hydroxyrosenoxid | 46,1 GC-Flächen-% |
| cis-Hydroxyrosenoxid | 27,3 GC-Flächen-% |
| 2-(2-Hydroxymethylpropyl)-4-methyltetrahydropyranol | 13,5 GC-Flächen-% |

### Beispiel 4: Herstellung von trans- und cis-Pyranol

In einem 300 ml Laborautoklaven wurden 20 g Hydroxyrosenoxid, gelöst in 80 ml Methanol in Gegenwart von 20 g eines Katalysators bestehend aus 50 Gew.-% NiO, 17 Gew.-% CuO, 30,5 Gew.-% ZrO₂ und 1,5 Gew.-% MoO₃ in Form von Tabletten mit einem Durchmesser und einer Höhe von jeweils von 3 mm bei einem Wasserstoffdruck von 30 bar und einer Temperatur von 95 °C unter kräftigem Rühren hydriert. Nach 4 h Reaktionsdauer wurde der Katalysator abfiltriert. Das erhaltene Reaktionsgemisch wurde zu den in Tabelle 1 angegeben Zeiten gaschromatographisch analysiert. Man erhielt die in Tabelle 1 angegebenen Ergebnisse (jeweils in GC-Flächen-%, Umsatz: 99% und Selektivität: 95%).

**Tabelle 1:**

| **Zeit (h)** | **0** | **2** | **4** |
|---|---|---|---|
| trans-Hydroxyrosenoxid | 55,5 | 4,5 | 0,4 |
| cis-Hydroxyrosenoxid | 36,4 | 2,9 | 0,3 |
| trans-Pyranol | 0 | 55,4 | 57,6 |
| cis-Pyranol | 0 | 34,0 | 36,3 |

## Patentansprüche

1. Verfahren zur Herstellung von 2-substituierten 4-Hydroxy-4-methyl-tetrahydropyranen der Formel (I) 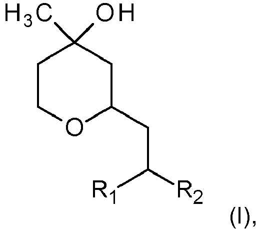 wobei der Rest
R₁ einen geradkettigen oder verzweigten Alkylrest mit 1 bis 5 Kohlenstoffatomen bedeutet, und
R₂ Wasserstoff oder einen geradkettigen oder verzweigten Alkylrest mit 1 bis 3 Kohlenstoffatomen bedeutet,
umfassend die Umsetzung von 3-Methylbut-3-en-1-ol der Formel (II) 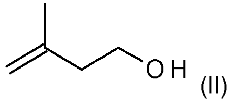 mit einem Aldehyd der Formel (III) 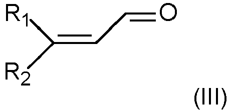 wobei der Rest R₁ und R₂ die gleichen Bedeutungen wie in Formel (I) angegeben haben, in Gegenwart von Wasser und in Gegenwart eines stark sauren Kationenaustauschers unter Bildung der Verbindung der Formel (IV), 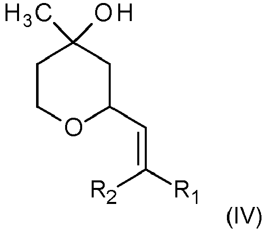 wobei die Reaktion in Gegenwart von 100 bis 200 mol-% Wasser durchgeführt wird, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe auf die Stoffmenge eines der beiden bezieht,
und Hydrierung in Gegenwart eines Katalysators zu einer Verbindung der Formel (I).

2. Verfahren nach Anspruch 1, **dadurch gekennzeichnet, dass** der Rest R₁ einen geradkettigen oder verzweigten Alkylrest mit 1 bis 3 Kohlenstoffatomen, insbesondere Methyl bedeutet.

3. Verfahren nach Anspruch 1, **dadurch gekennzeichnet, dass** der Rest R₂ Methyl oder Ethyl, insbesondere Methyl bedeutet.

4. Verfahren nach einem der Ansprüche 1 bis 3, **dadurch gekennzeichnet, dass** man Isoprenol und den Aldehyd der Formel (III) in einem molaren Verhältnis von 0,7 zu 1 bis 2 zu 1, insbesondere von 1 zu 1 bis 1,5 zu 1 einsetzt.

5. Verfahren nach einem der Ansprüche 1 bis 4, **dadurch gekennzeichnet, dass** man einen stark sauren, Sulfonsäuregruppen umfassenden Kationenaustauscher einsetzt.

6. Verfahren nach einem der Ansprüche 1 bis 5, **dadurch gekennzeichnet, dass** man mindestens einen stark sauren Kationenaustauscher in der H(+)-Form einsetzt, wobei der Ionenaustauscher ein Sulfonsäuregruppen aufweisendes Polymergerüst enthält und entweder gelförmig ist oder makroporöse Harze enthält.

7. Verfahren gemäss einem der Ansprüche 1 bis 6, **dadurch gekennzeichnet, dass** der Ionenaustauscher auf einem Polystyrolgerüst mit Sulfonsäuregruppen oder auf einem perfluorierten lonenaustauscherharz mit Sulfonsäuregruppen basiert.

8. Verfahren nach einem der Ansprüche 1 bis 7, **dadurch gekennzeichnet, dass** man die Umsetzung von Isoprenol mit einem Aldehyd der Formel (III) ohne Zusatz eines organischen Lösungsmittels durchführt.

9. Verfahren nach einem der Ansprüche 1 bis 8, **dadurch gekennzeichnet, dass** man die Umsetzung von Isoprenol mit einem Aldehyd der Formel (III) bei einer Temperatur im Bereich von 20 bis 80°C durchführt.

10. Verfahren gemäss einem der Ansprüche 1 bis 9, **dadurch gekennzeichnet, dass** die Hydrierung in Gegenwart von Wasserstoff und einem Katalysator, umfassend
- 30 bis 70 Gew.-%, bevorzugt 40 bis 60 Gew.-% sauerstoffhaltige Verbindungen des Nickels, berechnet als NiO,
- 15 bis 45 Gew.-%, bevorzugt 20 bis 40 Gew.-% sauerstoffhaltige Verbindungen des Zirkons, berechnet als ZrO₂,
- 5 bis 30 Gew.-%, bevorzugt 10 bis 25 Gew.-% sauerstoffhaltige Verbindungen des Kupfers, berechnet als CuO und
- 0,1 bis 10 Gew.-%, bevorzugt 0,5 bis 5 Gew.-% sauerstoffhaltige Verbindungen des Molybdäns, berechnet als MoO₃, gegebenenfalls
- 0 bis 10 Gew.-%, bevorzugt 0 bis 5 Gew.-% weitere Komponenten,
wobei sich die Angaben in Gew.-% auf den trockenen, nicht reduzierten Katalysator beziehen, durchgeführt wird.

11. Verfahren gemäss einem der Ansprüche 1 bis 10, **dadurch gekennzeichnet, dass** man die Hydrierung bei einer Temperatur im Bereich von 50 bis 130°C durchführt.

12. Verfahren gemäss einem der Ansprüche 1 bis 11, **dadurch gekennzeichnet, dass** man die Hydrierung bei einem Wasserstoffdruck im Bereich von 5 bis 200 bar absolut durchführt.

13. Verfahren nach einem der Ansprüche 1 bis 12, umfassend die Schritte
a. Bereitstellen eines den gewählten stark sauren Kationenaustauscher enthaltenden Durchflussrektors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (III) sowie Wasser mit dem stark sauren Kationenaustauscher im Durchflussreaktor unter Erhalt eines die gewünschten 2-substituierten 4-Hydroxy-4-methyl-tetrahydropyrane umfassenden Reaktionsgemisches,
d. kontinuierliche Hydrierung des die Verbindung der Formel (IV) umfassenden Reaktionsgemisches, und
e. kontinuierliches Austragen des Reaktionsgemisches aus dem Durchflussreaktor.

14. Verfahren nach einem der Ansprüche 1 bis 13 zur Herstellung von 2-substituierten 4-Hydroxy-4-methyl-tetrahydropyranen in Form von Gemischen des cis- Diastereomeren der Formeln (Ib) 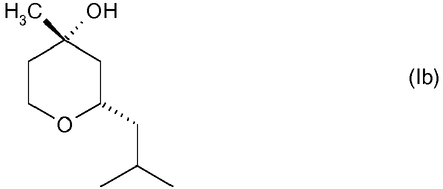 und des trans-Diastereomeren der Formel (Ic) 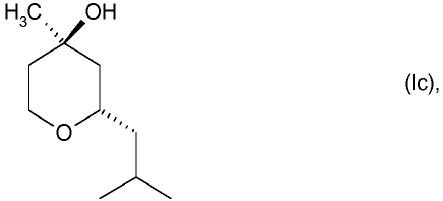 wobei das Diastereomerenverhältnis des cis-Diasteromeren der Formel (Ib) zum trans-Diastereomeren der Formel (Ic) 65 zu 35 bis 95 zu 5 beträgt.

## Claims

1. A process for the preparation of 2-substituted 4-hydroxy-4-methyltetrahydropyrans of the formula (I) 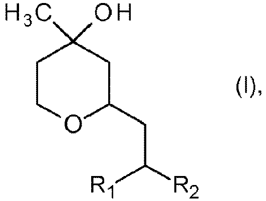 where the radical
R₁ is a straight-chain or branched alkyl radical having 1 to 5 carbon atoms, and
R₂ is hydrogen or a straight-chain or branched alkyl radical having 1 to 3 carbon atoms,
comprising the reaction of 3-methylbut-3-en-1-ol of the formula (II) 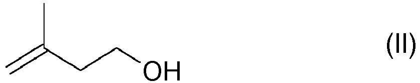 with an aldehyde of the formula (III) 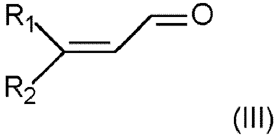 where the radicals R₁ and R₂ have the same meanings as given in formula (I), in the presence of water and in the presence of a strongly acidic cation exchanger to form the compound of the formula (IV), 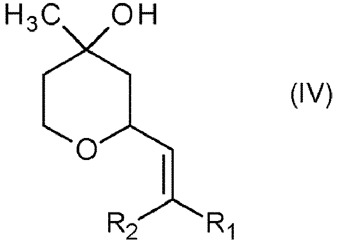 where the reaction is carried out in the presence of from 100 to 200 mol% of water, where the amount of water refers to the amount of the starting material isoprenol, optionally used in deficit, or to the aldehyde of the formula (III), or, in the case of the equimolar reaction of the two starting materials, to the quantitative amount of one of the two,
and hydrogenation in the presence of a catalyst to give a compound of the formula (I).

2. The process according to claim 1, wherein the radical R₁ is a straight-chain or branched alkyl radical having 1 to 3 carbon atoms, in particular methyl.

3. The process according to claim 1, wherein the radical R₂ is methyl or ethyl, in particular methyl.

4. The process according to any one of claims 1 to 3, wherein isoprenol and the aldehyde of the formula (III) are used in a molar ratio of from 0.7:1 to 2:1, in particular of from 1:1 to 1.5:1.

5. The process according to any one of claims 1 to 4, wherein a strongly acidic cation exchanger comprising sulfonic acid groups is used.

6. The process according to any one of claims 1 to 5, wherein at least one strongly acidic cation exchanger is used in the H(+) form, where the ion exchanger comprises a polymer backbone having sulfonic acid groups and is either gel-like or comprises macroporous resins.

7. The process according to any one of claims 1 to 6, wherein the ion exchanger is based on a polystyrene backbone with sulfonic acid groups or on a perfluorinated ion exchange resin with sulfonic acid groups.

8. The process according to any one of claims 1 to 7, wherein the reaction of isoprenol with an aldehyde of the formula (III) is carried out without addition of an organic solvent.

9. The process according to any one of claims 1 to 8, wherein the reaction of isoprenol with an aldehyde of the formula (III) is carried out at a temperature in the range from 20 to 80°C.

10. The process according to any one of claims 1 to 9, wherein the hydrogenation is carried out in the presence of hydrogen and a catalyst, comprising
- 30 to 70% by weight, preferably 40 to 60% by weight, of oxygen-containing compounds of nickel, calculated as NiO,
- 15 to 45% by weight, preferably 20 to 40% by weight, of oxygen-containing compounds of zirconium, calculated as ZrO₂,
- 5 to 30% by weight, preferably 10 to 25% by weight, of oxygen-containing compounds of copper, calculated as CuO and
- 0.1 to 10% by weight, preferably 0.5 to 5% by weight, of oxygen-containing compounds of molybdenum, calculated as MoO₃, optionally
- 0 to 10% by weight, preferably 0 to 5% by weight, of further components,
where the data in % by weight refer to the dry, nonreduced catalyst.

11. The process according to any one of claims 1 to 10, wherein the hydrogenation is carried out at a temperature in the range from 50 to 130°C.

12. The process according to any one of claims 1 to 11, wherein the hydrogenation is carried out at a hydrogen pressure in the range from 5 to 200 bar absolute.

13. The process according to any one of claims 1 to 12, comprising the steps
a. providing a flow reactor comprising the selected strongly acidic cation exchanger;
b. continuously introducing isoprenol, the aldehyde of the formula (III) and water into the flow reactor;
c. continuously bringing isoprenol, the aldehyde of the formula (III) and water into contact with the strongly acidic cation exchanger in the flow reactor to give a reaction mixture comprising the desired 2-substituted 4-hydroxy-4-methyltetrahydropyrans,
d. continuously hydrogenating the reaction mixture comprising the compound of the formula (IV), and
e. continuously discharging the reaction mixture from the flow reactor.

14. The process according to any one of claims 1 to 13 for the preparation of 2-substituted 4-hydroxy-4-methyltetrahydropyrans in the form of mixtures of the cis-diastereomer of the formula (Ib) 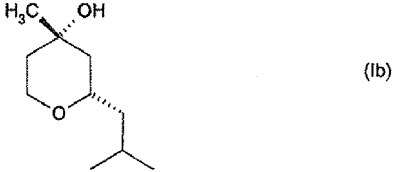 and of the trans-diastereomer of the formula (Ic) 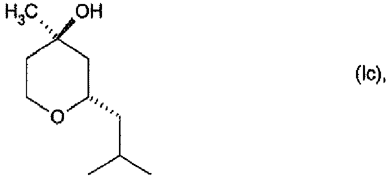 where the diastereomer ratio of the cis-diastereomer of the formula (Ib) to the trans-diastereomer of the formula (Ic) is 65:35 to 95:5.

## Revendications

1. Procédé de fabrication de 4-hydroxy-4-méthyl-tétrahydropyranes 2-substitués de formule (I) 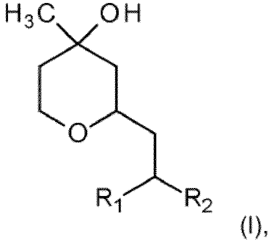 dans laquelle le radical
R₁ signifie un radical alkyle linéaire ou ramifié de 1 à 5 atomes de carbone, et
R₂ signifie hydrogène ou un radical alkyle linéaire ou ramifié de 1 à 3 atomes de carbone,
comprenant la mise en réaction de 3-méthylbut-3-én-1-ol de formule (II) 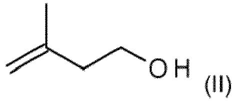 avec un aldéhyde de formule (III) 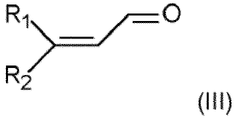 dans laquelle les radicaux R₁ et R₂ ont les mêmes significations que celles indiquées dans la formule (I), en présence d'eau et en présence d'un échangeur de cations fortement acide avec formation du composé de formule (IV) 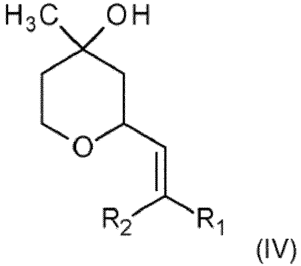 la réaction étant réalisée en présence de 100 à 200 % en moles d'eau, la quantité d'eau se rapportant à la quantité de la matière première isoprénol éventuellement utilisée en déficit ou de l'aldéhyde de formule (III) ou, dans le cas de la réaction équimolaire des deux matières premières, à la quantité de matière d'un des deux,
et l'hydrogénation en présence d'un catalyseur pour former un composé de formule (I).

2. Procédé selon la revendication 1, **caractérisé en ce que** le radical R₁ signifie un radical alkyle linéaire ou ramifié de 1 à 3 atomes de carbone, notamment méthyle.

3. Procédé selon la revendication 1, **caractérisé en ce que** le radical R₂ signifie méthyle ou éthyle, notamment méthyle.

4. Procédé selon l'une quelconque des revendications 1 à 3, **caractérisé en ce que** l'isoprénol et l'aldéhyde de formule (III) sont utilisés en un rapport molaire de 0,7 sur 1 à 2 sur 1, notamment de 1 sur 1 à 1,5 sur 1.

5. Procédé selon l'une quelconque des revendications 1 à 4, **caractérisé en ce qu'**un échangeur de cations fortement acide comprenant des groupes acide sulfonique est utilisé.

6. Procédé selon l'une quelconque des revendications 1 à 5, **caractérisé en ce qu'**au moins un échangeur de cations fortement acide sous la forme H(+) est utilisé, l'échangeur d'ions contenant un squelette polymère comprenant des groupes acide sulfonique, et étant sous la forme de gel ou contenant une résine macroporeuse.

7. Procédé selon l'une quelconque des revendications 1 à 6, **caractérisé en ce que** l'échangeur d'ions est à base d'un squelette polystyrène contenant des groupes acide sulfonique ou d'une résine échangeuse d'ions perfluorée contenant des groupes acide sulfonique.

8. Procédé selon l'une quelconque des revendications 1 à 7, **caractérisé en ce que** la réaction d'isoprénol avec un aldéhyde de formule (III) est réalisée sans ajout d'un solvant organique.

9. Procédé selon l'une quelconque des revendications 1 à 8, **caractérisé en ce que** la réaction d'isoprénol avec un aldéhyde de formule (III) est réalisée à une température dans la plage allant de 20 à 80 °C.

10. Procédé selon l'une quelconque des revendications 1 à 9, **caractérisé en ce que** l'hydrogénation est réalisée en présence d'hydrogène et d'un catalyseur, comprenant :
- 30 à 70 % en poids, de préférence 40 à 60 % en poids, de composés oxygénés de nickel, calculés en tant que NiO,
- 15 à 45 % en poids, de préférence 20 à 40 % en poids, de composés oxygénés de zirconium, calculés en tant que ZrO₂,
- 5 à 30 % en poids, de préférence 10 à 25 % en poids, de composés oxygénés de cuivre, calculés en tant que CuO, et
- 0,1 à 10 % en poids, de préférence 0,5 à 5 % en poids, de composés oxygénés de molybdène, calculés en tant que MoO₃, éventuellement
- 0 à 10 % en poids, de préférence 0 à 5 % en poids, d'autres composants,
les indications en % en poids se rapportant au catalyseur sec non réduit.

11. Procédé selon l'une quelconque des revendications 1 à 10, **caractérisé en ce que** l'hydrogénation est réalisée à une température dans la plage allant de 50 à 130 °C.

12. Procédé selon l'une quelconque des revendications 1 à 11, **caractérisé en ce que** l'hydrogénation est réalisée à une pression d'hydrogène dans la plage allant de 5 à 200 bar absolu.

13. Procédé selon l'une quelconque des revendications 1 à 12, comprenant les étapes suivantes :
a. la préparation d'un réacteur à circulation contenant l'échangeur de cations fortement acide choisi ;
b. l'introduction continue d'isoprénol, de l'aldéhyde de formule (III) et d'eau dans le réacteur à circulation ;
c. la mise en contact continue d'isoprénol, de l'aldéhyde de formule (III) et d'eau avec l'échangeur de cations fortement acide dans le réacteur à circulation pour obtenir un mélange réactionnel comprenant les 4-hydroxy-4-méthyl-tétrahydropyranes 2-substitués souhaités,
d. l'hydrogénation continue du mélange réactionnel comprenant le composé de formule (IV), et
e. le déchargement continu du mélange réactionnel du réacteur à circulation.

14. Procédé selon l'une quelconque des revendications 1 à 13, pour la fabrication de 4-hydroxy-4-méthyl-tétrahydropyranes 2-substitués sous la forme de mélanges du diastéréomère cis de formule (Ib) 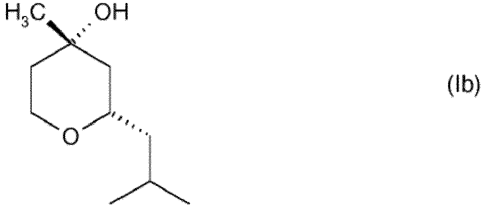 et du diastéréomère trans de formule (Ic) 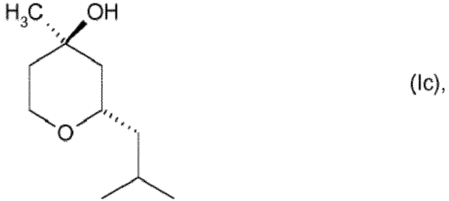 le rapport entre le diastéréomère cis de formule (Ib) et le diastéréomère trans de formule (Ic) étant de 65 sur 35 à 95 sur 5.
